# EUROPÄISCHE PATENTSCHRIFT

(11) **EP 4 327 762 B1**
(45) Veröffentlichungstag und Bekanntmachung des Hinweises auf die Patenterteilung: **01.10.2025**
(21) Anmeldenummer: 22192134.9
(22) Anmeldetag: 25.08.2022
(51) Int. Cl.: A61B 17/22, A61B 17/00

(54) **DRUCKWELLENGERÄT MIT DOPPELVENTILEINRICHTUNG**
PRESSURE WAVE DEVICE WITH DOUBLE VALVE DEVICE
APPAREIL À ONDES DE CHOC POURVU DE DISPOSITIF À DOUBLE SOUPAPE

(43) Veröffentlichungstag der Anmeldung: 28.02.2024
(73) Patentinhaber: STORZ MEDICAL AG, 8274 Tägerwilen (CH)
(72) Erfinder: STORZ, Rafael, 8280 Kreuzlingen (CH); BELAU, Markus, 78467 Konstanz (DE); KÜHL, Arvid, 8274 Tägerwilen (CH); HONSELL, Lukas, 78479 Reichenau (DE); GREMLICH, Felix, 8280 Kreuzlingen (CH); GLENZER, Thomas, 8280 Kreuzlingen (CH)
(74) Vertreter: Szynka Smorodin Patentanwälte Partnerschaft mbB

(56) Entgegenhaltungen:
- EP-A1- 2 529 679
- EP-B1- 2 213 273
- US-A1- 2009 326 425
- US-A1- 2011 275 965
- US-A1- 2014 350 438

## Beschreibung

Die Erfindung bezieht sich auf ein Gerät zur Behandlung des menschlichen oder tierischen Körpers mit mechanischen Druckwellen, welche durch Aufschlagen eines beschleunigten Projektils auf einen Applikator erzeugt werden.

Geräte dieses Typs sind seit einiger Zeit bekannt und zunehmend im Einsatz. Zur Behandlung des (menschlichen oder tierischen) Patienten dienen mechanische Druckwellen, die durch Auflegen eines Applikators auf den Körper des Patienten eingekoppelt werden und durch eine Kollision eines beschleunigten Projektils mit dem Applikator erzeugt werden. Dabei muss der Applikator nicht zwingend einstückig sein, sondern kann auch aus einer Mehrzahl verschiedener Teile oder Materialien zusammengesetzt sein.

Eine in der Praxis bewährte und vielfach beschriebene Technik zur Beschleunigung des Projektils ist pneumatisch. Dabei wird durch Beaufschlagung eines Volumens auf einer Seite des entlang einer Bewegungsstrecke, z. B. in einem Rohrstück, beweglichen Projektils ein pneumatischer Überdruck eingekoppelt.

Im Stand der Technik wird hierfür ein Schaltventil benutzt, das an eine Pneumatikversorgung, insbesondere einen Kompressor mit regelbarem Ausgangsdruck, angeschlossen ist und dessen Puls das Projektil von einem zu dem Applikator distalen Ende der Bewegungsstrecke aus hin zu dem Applikator beschleunigt. Die pneumatische Beaufschlagung wird bei Erreichen des proximalen Endes der Bewegungsstrecke ausgeschaltet, also mit dem Aufschlagen auf dem Applikator.

Die Rückbewegung erfolgt im Stand der Technik mithilfe einer Gegendruckkammer, also eines Speichervolumens, in das das zu dem Applikator hin bewegte Projektil gewissermaßen die vor ihm befindliche Luft verdrängt, womit es dieses Speichervolumen quasi aufpumpt.

In der vorveröffentlichten, allerdings wegen mangelnder Ausführbarkeit im Einspruchsbeschwerdeverfahren widerrufenen EP 2 181 730 B1 wird neben einer nicht näher ausgeführten Steuerung der Öffnungszeit des Schaltventils für die Beschleunigung auch eine gezielte Druckbegrenzung in dieser Gegendruckkammer diskutiert. Außerdem erwähnt dieses Dokument den Einsatz eines zweiten Schaltventils für eine Rückführung des Projektils in die distale Ausgangslage nach der Beaufschlagung durch das erste Schaltventil.

Die US 2014/350438 A1 beschreibt ein ballistisches Druckwellengerät nach dem Oberbegriff des Anspruchs 1, das zur Erzeugung besonders intensiver Wellen zur Zerkleinerung von Körperkonkrementen ausgelegt ist. Zur Rückführung des Projektils wird unter anderem eine pneumatische Lösung mit einem zweiten Ventil vorgeschlagen.

Die US 2009/326425 A1 beschreibt ein ballistisches Druckwellengerät, dessen pneumatisches System dazu ausgelegt ist, dass in Folge eines Beschleunigungsvorgangs des Projektils mindestens zwei Kollisionen stattfinden.

Die DE 20 2010 009 899 U1 beschreibt ein elektromagnetisches Druckwellengerät, das zur Rückführung des Projektils und Vermeidung von Schäden einer Rückstellfeder einen zweiten Elektromagneten vorschlägt.

Der vorliegenden Erfindung liegt die Aufgabe zugrunde, auf dieser Grundlage ein hinsichtlich der Hin- und Rückbewegung des Projektils verbessertes Gerät des beschriebenen Typs mit pneumatischer Einrichtung zur Projektilbewegung anzugeben.

Zur Lösung dieser Aufgabe wird das Gerät gemäß Anspruch 1 vorgeschlagen. Bevorzugte Ausgestaltungen sind Gegenstand der abhängigen Ansprüche.

Dementsprechend weist das erfindungsgemäße Gerät als Teil seiner pneumatischen Einrichtung eine Doppelventileinrichtung zur Beaufschlagung des Projektils in beide Richtungen, also zu dem Applikator hin und umgekehrt von ihm weg in der Rückrichtung, auf, also z. B. die Kombination eines ersten und eines zweiten Ventils. Die Zeitphasen, in denen das Projektil so pneumatisch beaufschlagt wird, dass es sich in der Hinrichtung bewegt, also z. B. die Einschaltphase eines ersten Ventils, wird im Folgenden als erste Einschaltzeit bezeichnet und umgekehrt als zweite Einschaltzeit eine Zeitphase einer umgekehrten Beaufschlagung des Projektils.

Erfindungsgemäß soll das Gerät dazu ausgelegt sein (also insbesondere eine darin vorhandene Steuereinrichtung so ausgelegt sein), eine zweite Einschaltzeit schon zu beenden, bevor das Projektil vollständig zurückbewegt worden ist, also nach einer nur teilweisen Rückbewegung. Außerdem soll die erste Einschaltzeit auch bereits vor der vollständigen Rückkehr und damit ebenfalls nach einer nur teilweisen Rückbewegung beginnen, allerdings nicht zwingend gleichzeitig mit dem Ende der zweiten Einschaltzeit. Insgesamt kann und soll damit erreicht werden, dass sich das Projektil (jedenfalls in bestimmten Steuerungszuständen) gar nicht mehr vollständig zurückbewegt, sondern bereits nach einem Teil der Bewegungsstrecke und vor deren Ende (mit dem Anschlag) seine Bewegungsstrecke umkehrt.

Erfindungsgemäß wird also durch ein frühzeitiges Ende der zweiten Einschaltzeit und ein Einsetzen der (folgenden) ersten Einschaltzeit noch vor Erreichen des distalen Endes der Bewegungsstrecke eine Verkürzung der Bewegungsstrecke auf eine effektive Länge gegenüber der geometrisch möglichen Bewegungsstrecke erreicht.

Daraus ergeben sich verschiedene Möglichkeiten und Vorteile, die je nach Anwendungsfall genutzt werden können. Z. B. kann unabhängig von dem genutzten pneumatischen Druck die Aufschlaggeschwindigkeit des Projektils auf dem Applikator variiert und insbesondere gesteuert werden. Je kürzer nämlich die effektive Beschleunigungsstrecke (bei angenommen konstantem beschleunigenden Druck), umso kleiner die Aufschlaggeschwindigkeit. Insoweit bietet die Erfindung einen weiteren Freiheitsgrad.

Insbesondere lassen sich durch die Verkürzung der effektiven Beschleunigungsstrecke besonders kleine Aufschlaggeschwindigkeiten realisieren, die allein durch eine Druckverringerung nicht zugänglich wären. Erfahrungsgemäß setzen die hier behandelten pneumatischen Antriebe einen gewissen Mindestdruck voraus, um das Projektil überhaupt definiert bewegen zu können. Das kann z. B. Resultat einer Haftreibung zwischen dem Projektil und den Innenmantelflächen des ihn führenden Rohrstücks sein. Erfindungsgemäß kann nun bei einem für einen sichere und definierte Projektilbewegung ausreichenden Druck durch Streckenverkürzung die Geschwindigkeit noch weiter abgesenkt werden.

Ein zusätzlicher Vorteil kann darin bestehen, bei einer gegebenen gewünschten Aufschlaggeschwindigkeit gegenüber der konventionellen Vorgehensweise (mit Ausnutzung der gesamten geometrisch gegebenen Bewegungsstrecke) die Betriebsfrequenz erhöhen zu können. Wenn nämlich die gewünschte Aufschlaggeschwindigkeit mit einem kleineren als dem verfügbaren Druck erzielbar ist und statt einer Verringerung dieses Drucks (etwa durch einen Druckminderer oder auch durch eine Steuerung der Druckquelle) in der beschriebenen Weise die effektive Bewegungsstrecke verkürzt wird, so benötigt das Projektil für die Hinbewegung und außerdem auch für die Rückbewegung jeweils weniger Zeit.

Weiter oben wurde die Kombination zweier Schaltventile angesprochen, die eine Möglichkeit für eine erfindungsgemäß vorgesehene Doppelventileinrichtung darstellt. Bei dieser Variante sind die beiden Ventile (vorzugsweise unabhängig voneinander) von der Steuereinrichtung ansteuerbar. Alternativ kann aber auch ein einheitliches Ventil verwendet werden, das hier als "Kombinationsventil" bezeichnet wird und das abhängig von der Ansteuerung durch die Steuereinrichtung mindestens zwei Schaltzustände aufweist, nämlich einen ersten zur Beaufschlagung des Projektils in Richtung zu dem Applikator hin und einen zweiten zur Beaufschlagung in der Rückrichtung. Während das Kombinationsventil in dem ersten Schaltzustand ist, ist also eine erste Ventilöffnungszeit gegeben, und im zweiten Schaltzustand dementsprechend eine zweite Ventilöffnungszeit.

In diesen beiden Schaltzuständen wird der jeweils im anderen Schaltzustand zu beaufschlagende pneumatische Anschluss durch das Kombinationsventil vorzugsweise belüftet, sodass also z. B. bei der Hinbewegung auf der zu dem Applikator proximalen Seite des Projektils ungefähr Umgebungsdruck herrscht und im Unterschied zu dem konventionellen Vorgehen mit einer Gegendruckkammer kein von Kollision zu Kollision zunehmender Staudruck.

Auch bei dem Einsatz zweier separater Ventile ist vorzugsweise mindestens eines der beiden Ventile ein Zweiwegeventil, das dementsprechend eine Belüftung durchführt, sofern es nicht zur Beaufschlagung mit dem pneumatischen Druck geschaltet ist.

Weiter oben wurde bereits die Möglichkeit der Steuerung der Aufschlaggeschwindigkeit des Projektils mittels des tatsächlich verwendeten Teils der möglichen Bewegungsstrecke angesprochen. Eine zusätzliche Möglichkeit besteht darin, bei der Beschleunigung des Projektils in Richtung zu dem Applikator hin eine erste Einschaltzeit (verantwortlich für die Druckbeaufschlagung des Projektils in der Hinrichtung) mit einer darauffolgenden oder vorausgehenden zweiten Einschaltzeit (verantwortlich für die Druckbeaufschlagung in der entgegengesetzten Richtung) überlappen zu lassen. Während des Zeitraums einer solchen Überlappung, z. B. direkt vor dem Aufschlagen des Projektils auf den Applikator, kompensieren sich die pneumatischen Drücke auf den beiden Seiten des Projektils zumindest weitgehend, sodass das Projektil gewissermaßen kräftefrei bleibt (von Reibung abgesehen). Insoweit kann in dieser Form zusätzlich Einfluss auf die Aufschlaggeschwindigkeit genommen werden, und zwar ohne den anstehenden pneumatischen Druck in seiner Höhe zu verändern.

Für das zuvor beschriebene Kombinationsventil bedeutet das einen zusätzlichen Schaltzustand, in dem beidseits eine Druckbeaufschlagung stattfindet.

Eine Kombination beider Möglichkeiten, also der Nutzung nur eines Teils oder sogar Variation des verwendeten Teils der Bewegungsstrecke einerseits und andererseits einer Überlappung der Einschaltzeiten der beiden Ventile, kann durchaus sinnvoll sein. Z. B. lässt sich mit einem Überlappzeitraum vor dem Aufschlagen des Projektils auf den Applikator eine Begrenzung der Ausschlaggeschwindigkeit erzielen, wobei (im Vergleich zu einer Beschleunigung über die gesamte Bewegungsstrecke) die gemittelte Geschwindigkeit trotzdem relativ groß bleibt. Wenn nämlich zunächst beschleunigt und dann während des Überlappzeitraums nicht mehr beschleunigt wird, wird vergleichsweise früh ein höheres Geschwindigkeitsniveau erreicht als bei einer durchgehenden Beschleunigung bis zur Kollision (gleiche Kollisionsgeschwindigkeit vorausgesetzt). Wenn also z. B. mit einem minimalen Beschleunigungsdruck einerseits auch eine besonders kleine Aufschlaggeschwindigkeit realisiert werden soll (wie oben erläutert), ohne dabei aber die Frequenz (zu sehr) zu erhöhen, kann z. B. in der Anfangsphase der Hinbewegung in einer ersten Einschaltzeit eine Beschleunigung stattfinden und diese dann durch eine Überlappung der beiden Einschaltzeiten beendet werden. Es bleibt dann (ganz ungefähr) bei der erreichten Projektilgeschwindigkeit, es vergeht aber mehr Zeit bis zur Kollision, gewissermaßen durch ein Stück für die Beschleunigung nicht genutzte Wegstrecke. Also bietet die Überlappzeit im Rahmen der Erfindung einen zusätzlichen Freiheitsgrad.

Natürlich können dabei Steuerungszustände mit verschiedenen solchen Überlappzeiten existieren einschließlich einer Überlappzeit Null. Außerdem kann es grundsätzlich auch nach der Kollision eine Überlappzeit geben. Wenn ausnahmsweise die vollständige Bewegungsstrecke genutzt wird, kann es im Prinzip auch eine Überlappzeit oder einen Teil einer Überlappzeit nach der Bewegungsumkehr am distalen Ende der Bewegungsstrecke geben. Bei einer repetitiven Bewegung kann das Projektil an diesem Ende quasi reflektiert werden und schon infolge des Impulsaustauschs die Bewegung in Hinrichtung starten.

Außerdem kann bei gegebenem pneumatischem Druck eine allzu heftige Rückbeschleunigung und Kollision des Projektils mit einem Anschlag an einem von dem Applikator distalen Ende der Bewegungsstrecke vermieden werden, wenn bei dem betreffenden Steuerungszustand (ausnahmsweise) die volle Bewegungsstrecke genutzt wird. Außerdem kann durch einen Überlappzeitraum nach der Kollision die Zeit für die Rückbewegung verlängert werden, ohne damit die faktisch genutzte Bewegungsstrecke zu vergrößern, falls dies mit Rücksicht auf eine bestimmte Kombination aus Frequenz und Aufschlaggeschwindigkeit (bei gegebenem Druck) gewünscht ist.

Insbesondere kann bei der Variation einer Überlappzeit der beiden Einschaltzeiten die zweite Einschaltzeit hinsichtlich ihrer Dauer und/oder ihres Beginns variiert werden, wobei die erste Einschaltzeit dabei vorzugsweise konstant bleiben kann.

Eine weitere Möglichkeit für andere Steuerungszustände und in diesem Sinn gewissermaßen das Gegenteil der beschriebenen Überlappzeit ist eine Abstandszeit zwischen der ersten und der zweiten Einschaltzeit, oder umgekehrt, wobei in diesem Sinn hier eine Abstandszeit zwischen einer ersten und der darauffolgenden zweiten Einschaltzeit gemeint ist (und nicht umgekehrt, also im Sinn der Folge einer ersten Einschaltzeit auf eine zweite in der Umgebung eines distalen Umkehrpunkts der Projektilbewegung).

Eine solche Abstandszeit führt in ähnlicher Weise wie die Überlappzeit zu einem quasi kräftefreien Bewegungsmoment des Projektils und kann insoweit ähnlich eingesetzt werden, wobei bei einem Gerät auch Steuerungszustände mit Überlappzeit und andere Steuerungszustände mit Abstandszeit (und möglicherweise auch noch solche mit unmittelbarer Berührung der ersten und der zweiten Einschaltzeit im Sinne einer Abstandszeit Null) existieren können.

Insbesondere kann also bei einer Abstandszeit die Projektilgeschwindigkeit beim Aufschlagen verringert werden. Außerdem kann bei der Rückbewegung eine gewisse Verlangsamung erreicht werden, wenn die oder ein Teil der Abstandszeit nach dem Aufschlagen liegt. Das wurde oben bereits in ähnlicher Form für die Überlappzeit erläutert.

Bei den vorstehenden Erklärungen wurde wiederholt darauf abgestellt, den pneumatischen Druck nicht zwingend verändern zu müssen. Es ist in der Tat bevorzugt, diesen Druck während des Betriebs und in verschiedenen Steuerungszuständen unverändert aufrecht zu erhalten.

Die pneumatische Einrichtung kann im einfachsten Fall einen Anschluss zur Versorgung aus einem pneumatischen Leitungsnetz, z. B. in einem Krankenhaus, oder aus einer Druckgasflasche aufweisen. Bevorzugt ist jedoch ein Pneumatikkompressor, mit dem das erfindungsgemäße Gerät örtlich unabhängig und im Vergleich zu einer Druckgasflasche mobiler ist. An sich sind Pneumatikkompressoren im Zusammenhang mit solchen Geräten vorbekannt. Die Erfindung bietet jedoch den oben erwähnten Aspekt, bei verschiedenen Steuerungszuständen mit unterschiedlichen Aufschlaggeschwindigkeiten des Projektils nicht zwingend den Versorgungsdruck ändern zu müssen. In anderen Worten kann der Kompressor in solchen verschiedenen Steuerungszuständen bei gleicher Drehzahl laufen.

Natürlich kann dadurch zunächst einmal die Ansteuerung des Kompressors vereinfacht werden, insbesondere wenn dieser grundsätzlich im eingeschalteten Zustand immer bei der gleichen Drehzahl läuft. Ferner kann der Kompressor dabei in der Nähe seines oder bei seinem Effizienzmaximum (hinsichtlich der Drehzahl) betrieben werden. Überdies ist es möglich, Geräuschminderungsmaßnahmen, z. B. eine dämpfende Lagerung des Kompressors oder eine geräuschdämmende Umhüllung, auf das Schwingungsverhalten des Kompressors bei der einen gleichen Drehzahl abzustimmen.

Eine besondere Gestaltungsmöglichkeit der Erfindung liegt darin begründet, allein durch Veränderung von Ventilöffnungszeitpunkten oder Ventilöffnungszeitdauern die Stoßphysik zwischen Projektil und Applikator direkt und schnell beeinflussen zu können, insbesondere die Aufschlaggeschwindigkeit. Im Vergleich zu einer Veränderung des Versorgungsdrucks ist diese Einflussnahmemöglichkeit besonders schnell, sodass in einem iterativen Betriebszustand prinzipiell von einem Aufschlagprozess zum nächsten die Aufschlaggeschwindigkeit und/oder die Zeitdauer der kombinierten Hin- und Rückbewegung, gewissermaßen also eine momentane Frequenz, verändert werden können. Eine so schnelle und freie Einflussnahme erlaubt der Stand der Technik nicht.

Typische Aufschlaggeschwindigkeiten liegen dabei, aber auch bei sich weniger schnell oder nicht verändernden Bedingungen, im Bereich zwischen 2 m/s und 30 m/s. Für die Stoßphysik ist vor allem der Aufschlagimpuls wichtig, der bei typischen Projektilmassen zwischen 1 g und 10 g somit in einem Bereich zwischen 2 gm/s und 300 gm/s, vorzugsweise zwischen 10 gm/s und 150 gm/s liegen kann.

Bei einer besonderen Ausgestaltung weist das Gerät eine Messeinrichtung auf, mit der der Durchtritt des Projektils an einer Stelle seiner Bewegungsstrecke gemessen werden kann. Diese Messeinrichtung ist mit der Steuereinrichtung gekoppelt. Damit kann in solcher Form z. B der Durchtritt des Projektils kurz vor dem Aufschlagen oder quasi beim Aufschlagen auf den Applikator erfasst werden, sodass die Einschaltzeiten dementsprechend (insbesondere hinsichtlich ihres Anfangs und ihres Endes) auf den Zeitpunkt des Aufschlagens abgestimmt werden können.

Eine solche Erfassung kann z. B. optisch erfolgen, etwa durch eine Lichtschranke oder dergleichen, vorzugsweise aber induktiv unter Verwendung einer Messspule. Diese kann durch einen Restmagnetismus des Projektils oder rein induktiv (durch Veränderung der Streuinduktivität) das Projektil erfassen.

Im Folgenden wird die Erfindung anhand von Ausführungsbeispielen näher erläutert, wobei die einzelnen Merkmale im Rahmen der Ansprüche auch in anderer Kombination erfindungswesentlich sein können.

Im Einzelnen zeigt
- Figur 1: eine perspektivische Darstellung eines erfindungsgemäßen Geräts, wobei ein mittlerer Gehäuseteil der Übersichtlichkeit halber weggelassen ist;
- Figur 2: einen Längsschnitt durch das Gerät aus Figur 1 in gegenüber Figur 1 rechts-links-vertauschter Lage;
- Figur 3: ein schematisches Diagramm des Handstücks mit einem zugehörigen Basisgerät;
- Figur 4: eine Folge schematischer Zeitverlaufsdiagramme 4a) bis d) zur Erläuterung der Funktionsweise;
- Figur 5: eine schematische Darstellung eines Kombinationsventils zur Erläuterung eines alternativen Ausführungsbeispiels zu den Figuren 1 und 2.

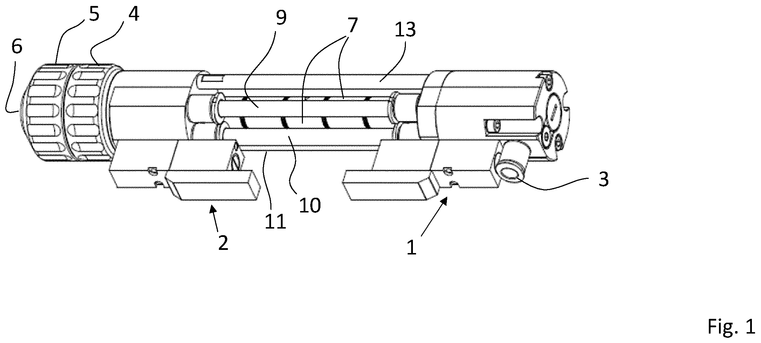

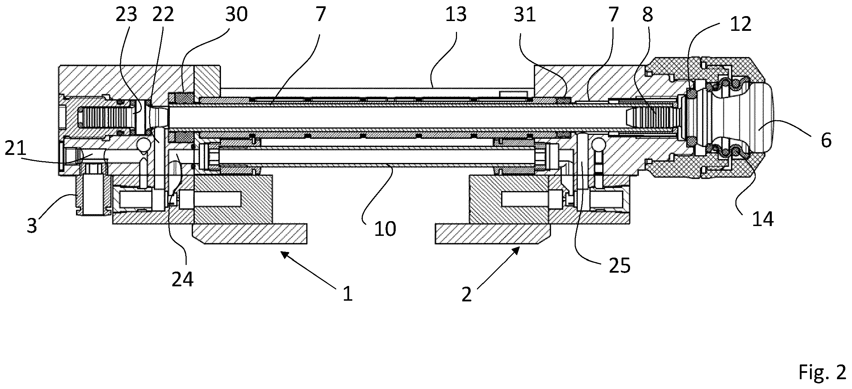

Figur 1 zeigt ein Handstück eines erfindungsgemäßen Geräts in perspektivischer Ansicht mit nach vorne-links weisenden pneumatischen Ventilen, nämlich einem ersten Ventil 1 und einem zweiten Ventil 2. Rechts sieht man einen pneumatischen Versorgungsanschluss 3 und links zwei zur leichteren Handhabung jeweils außen geriffelte Schraubringe 4 und 5 zur Halterung des im Folgenden noch näher erläuterten Applikators 6. Dieser ist in Figur 1 ganz links mit seiner patientenzugewandten Oberfläche gerade noch zu sehen und im Übrigen in Figur 2 gezeigt. Er könnte auch mehrteilig aufgebaut sein.

Im mittleren Bereich des Geräts aus Figur 1 erkennt man eine Mehrzahl in Querrichtung laufender Röhren, wobei die mittlere mit dem Bezugszeichen 7 das in Figur 2 im Schnitt erkennbare Projektil 8 enthält und führt. Davor sieht man zwei parallele pneumatische Verbindungsrohrleitungen 9 und 10 zwischen den beiden Ventilen 1 und 2, wobei die Rohrleitung 9 zum Zuführen einer Druckbeaufschlagung zu dem zweiten Ventil 2 und die Rohrleitung 10 umgekehrt zur Entlüftung dieses zweiten Ventils 2 über einen bei dem ersten Ventil 1 vorgesehenen Auslass dient. Diese Mehrzahl Rohre ist bei diesem Ausführungsbeispiel von einer in Figur 1 durch die Linie unter der Rohrleitung 10 und die beiden Linien über dem Projektilführungsrohr 7 dargestellten Gehäuseabdeckung 11 umgeben. Diese Gehäuseabdeckung 11 läuft in Figur 1 im hinteren Bereich und umfasst nur einen Teil des Umfangs. Sie ist an ihren jeweiligen axialen Kanten ähnlich einer Bördelung durch ein verrundetes Umschlagen nach innen griffgünstig gestaltet, was in Figur 1 bei der oberen Kante angedeutet ist. Die Gehäuseabdeckung 11 kann also bei der praktischen Handhabung als Griff dienen. Der Abstandshalter 13 stabilisiert den Aufbau und verbindet die beiden Enden des Handstücks mechanisch.

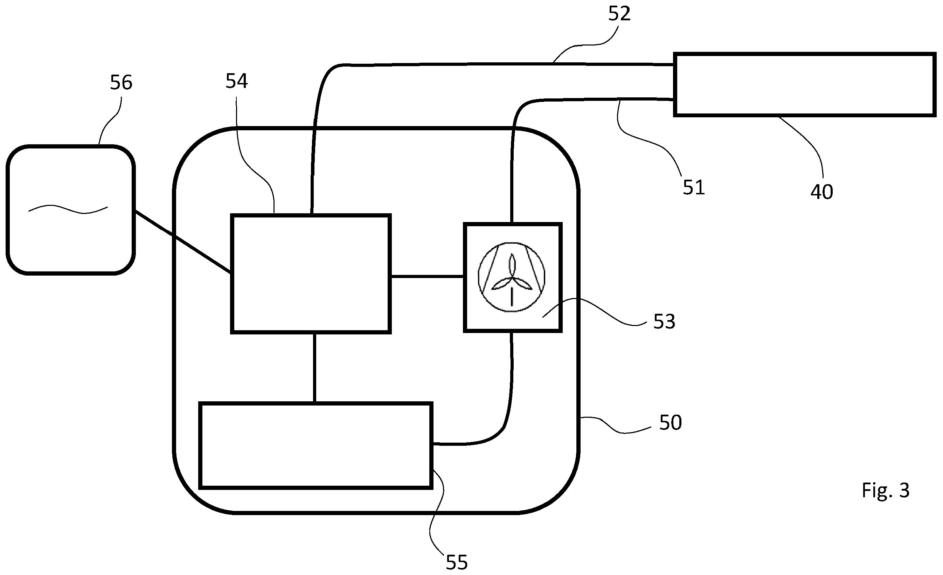

Eine von einem Pneumatikkompressor zu dem Gerät führende flexible Druckluftzuleitung (vgl. 51 in Figur 3) ist hier nicht eingezeichnet und an den bereits erwähnten Anschluss 3 anzuschließen. Analog ist eine elektronische Steuerleitung (52 in Figur 3) von einer externen Steuerung zu den Ventilen 1 und 2 nicht eingezeichnet, die mit der Druckluftzuleitung einheitlich ausgeführt sein kann.

Figur 2 zeigt einen Längsschnitt entlang einer gedachten Mittellängsachse der bereits erwähnten Zylinderform des Gesamtgeräts, die gleichzeitig eine Mittellängsachse des Projektilführungsrohrs 7 ist. Zur Veranschaulichung der Dimensionen: Die Länge des Projektilführungsrohrs 7 beträgt bei diesem Ausführungsbeispiel 145.5 mm und die übrige Darstellung in Figur 2 ist maßstäblich. In diesem Projektilführungsrohr ist das Projektil 8 in Figur 2 rechts eingezeichnet und damit in Anlage an dem Applikator 6, der von dem beschriebenen Schraubring 4 und 5 in einer an sich bekannten Weise gehalten ist. Dabei ist der Applikator 6 mit einem balgartigen Elastomerring 14 in Axialrichtung elastisch gelagert und mit einem weiteren Elastomerring 12 pneumatisch abgedichtet. Alternativ ist auch ein Geräteaufbau hinsichtlich des Applikators 6 und seiner Halterung und Abdichtung gemäß z. B. der EP 2 529 679 (auch unabhängig von der dort gezeigten Kappe) oder der EP 2 095 843 (auch unabhängig von dem dort thematisierten Material Keramik) möglich und bevorzugt.

Figur 2 zeigt links einen inneren Kanal 21, der den Pneumatikanschluss 3 mit dem ersten Ventil 1 verbindet. Das erste Ventil 1 kann demzufolge einen an dem Pneumatikanschluss 3 anstehenden Versorgungsdruck steuerungsabhängig auf einen radialen Kanal 22 schalten, der unter einem Dämpferelement 23 mündet und damit an das Innenvolumen des Projektilführungsrohrs 7 angeschlossen ist. Über diesen Kanal 22 wird also das Projektil während einer ersten Einschaltzeit in Richtung zu dem Applikator 6 hin beaufschlagt bzw. beschleunigt. Unabhängig davon wird der pneumatische Versorgungsdruck über den Kanal 24 und das Rohr 10 an das zweite Ventil 2 weitergegeben.

In der zweiten alternativen Schaltstellung werden der Kanal 22 und damit auch das Innenvolumen des Projektilführungsrohrs 7 zwischen dem distalen Ende (in Figur 2 links) und dem Projektil 8 belüftet.

In dem zweiten Ventil 2, das grundsätzlich spiegelsymmetrisch zu dem ersten Ventil 1 aufgebaut ist, kann der über das Rohr 10 anliegende pneumatische Versorgungsdruck alternativ über den Kanal 25 radial nach oben gegeben werden zu einem das Projektilführungsrohr 7 umgebenden Volumen (in Figur 2 als Schlitz über und unter dem Rohr 7 zu erkennen), das von dem Anschluss des Kanals 25 aus nach rechts, also in Richtung zu dem Applikator 6, führt und dort zwischen dem Applikator 6 und dem zu ihm proximalen Ende des Projektilführungsrohrs 7 an das Innenvolumen des Projektilführungsrohrs 7 angeschlossen ist (von der in Figur 2 gezeichneten Anwesenheit des Projektils 8 dort abgesehen). Über den Kanal 25 kann also der pneumatische Versorgungsdruck schaltbar an das Innenvolumen des Projektilführungsrohrs 7 zwischen dem Applikator 6 und dem Projektil 8 angelegt werden. Dabei ist allerdings bei diesem Beispiel die pneumatische Verbindung in Folge eines kleineren effektiven Öffnungsquerschnitts etwas schlechter, als an der entgegengesetzten Seite des Projektilführungsrohrs 7, so dass sich hier bei größeren Luftströmungsgeschwindigkeiten (höhere Frequenzen, größere Drücke) früher oder stärker Verzögerungen bemerkbar machen.

Alternativ kann das zweite Ventil 2 in der anderen Schaltstellung den Anschluss des Innenvolumens des Rohrs 10 an ihm sperren und den Kanal 25 und damit das Innenvolumen des Projektilführungsrohrs 7 rechts vom Projektil 8 belüften, also über eine pneumatisch gut leitfähige Verbindung an die Außenatmosphäre anschließen.

Die beiden Ventile 1 und 2 können mithin das Projektil von beiden Seiten mit pneumatischem Druck beaufschlagen, und zwar unabhängig voneinander und damit gleichzeitig oder wechselseitig oder können das Innere des Projektilführungsrohrs 7 beiderseits belüften.

Das Bezugszeichen 30 in Figur 2 bezeichnet einen ringförmigen Permanentmagneten am gegenüber dem Applikator 6 distalen Ende der (mit der Länge des Projektilführungsrohrs 7 zusammenfallenden) Bewegungsstrecke des Projektils 8. Mit diesem Magneten 30 kann das aus ferromagnetischem Material aufgebaute Projektil 8 an diesem distalen Ende der Bewegungsstrecke leicht fixiert werden. Durch einseitige Druckbeaufschlagung mittels des Ventils 2 kann das Projektil ferner in diese Position zurückgeführt und optional auch zusätzlich dort gehalten werden, insbesondere bei Betriebsbeginn oder bei nicht ferromagnetischem Projektil. Insoweit kann der Permanentmagnet 30 optional auch weggelassen werden, vor allem dann, wenn die im weiteren Verlauf noch erläuterten Reflexionen an diesem distalen Ende der Bewegungsstrecke auch bei kleinen Aufprallgeschwindigkeiten des Projektils 8 dort ermöglicht werden sollen.

Mit 31 ist eine Stelle beziffert, an der man mit einer Messspule den Durchtritt des Projektils 8 durch die entsprechende Stelle der Bewegungsstrecke erfassen könnte, wobei diese Stelle relativ nah an dem Applikator 6 liegt. Im einfachsten Fall nutzt man hier einen leichten Restmagnetismus des Projektils 8 aus, aber man könnte natürlich auch die Veränderung der Induktivität der Spule 31 wechselstromtechnisch erfassen und auswerten. Man kann die Kollision des Projektils 8 mit dem Applikator 6 auch durch die Verwendung eines Mikrophons oder Bewegungssensors im Versuchsaufbau ermitteln. Außerdem kann man im Versuchsaufbau z. B. mit zwei kurz vor dem Applikator 6 positionierten Lichtschranken die Aufprallgeschwindigkeit des Projektils 8 ermitteln.

Figur 3 zeigt ein Blockschema mit dem in den Figuren 1 und 2 dargestellten Gerät rechts oben, und zwar mit dem Bezugszeichen 40 summarisch bezeichnet. Dies Gerät 40 ist ein in der Hand zu haltendes mobiles Handstück, wie es von einschlägigen Geräten aus dem Stand der Technik an sich bereits bekannt ist. Es ist über zwei Leitungen 51 und 52 an eine Basisstation 50 angeschlossen, welche einen Pneumatikkompressor 53 und eine Steuerung 54 enthält. Der Kompressor 53 ist über die Leitung 51, nämlich eine pneumatische flexible Schlauchleitung, mit dem Handgerät 40 verbunden und die Steuerung 54 über die (optional mit der Leitung 51 integrierte) elektrische Leitung 52, über die die Steuerung auf die bereits erwähnten beiden Ventile 1 und 2 zugreifen und diese mit Strom versorgen kann. Über die Leitung 52 kann außerdem eine Kommunikation mit dem Handstück 40 erfolgen, insbesondere wenn dort zusätzlich eine Steuerung bzw. ein Teil der Steuerung vorgesehen ist.

Die Steuerung 54 steuert im Übrigen auch den Kompressor 53 hinsichtlich seiner Drehzahl und natürlich des Ein- und Ausschaltens und wird ihrerseits, genauso wie der Kompressor 53, von einem Netzgerät 55 leistungsversorgt. In dem Kompressor 53 kann aber auch eine die Drehzahl beeinflussende Druckregelung oder ein Regelventil integriert sein. Außerdem ist die Steuerung 54 an ein Display 56 angeschlossen, das in dem Basisgerät 50 eingebaut oder auch separat davon implementiert sein kann. Das Basisgerät 50 wird über ein berührungsempfindliches Display 56 und/oder über eine hier nicht dargestellte Anordnung von Tasten bedient.

Der Benutzer kann also die Funktion des Geräts 40 anhand solcher Tasten und jedenfalls anhand des Displays 56 steuern, wobei die Steuerung 54 insbesondere die Öffnungs- und Schließzeiten und damit auch die Öffnungsdauern der beiden Ventile 1 und 2 vorgibt. Teilaufgaben der Steuerung 54 können auch im Handstück 40 integriert sein, besonders was die Ansteuerung der Ventile 1 und 2 betrifft.

Zum Grundverständnis der Ansteuerung der beiden Ventile kann verwiesen werden auf das ältere Patent EP 2 213 273 B1. Das Ausführungsbeispiel darin entspricht hinsichtlich der Dimensionierung insbesondere des Projektilführungsrohrs und des Projektils weitgehend den obigen Erläuterungen und den Figuren 1 und 2 mit Ausnahme der Existenz des zweiten Ventils 2 und des Wegfalls der Gegendruckkammer. Außerdem wird in dem zitierten Ausführungsbeispiel von einer bestimmten Ventilöffnungszeit des dort einzigen Ventils bei einem bestimmten Druck ausgegangen, wohingegen die Projektilbeschleunigung im vorliegenden Fall durch den Anteil der ersten Ventilöffnungszeit auch außerhalb der Überlappzeit und damit auch bei einem konstanten Druck variabel erfolgt. Für die folgenden Erläuterungen kann beispielhaft von einem Druck von 4 bar ausgegangen werden.

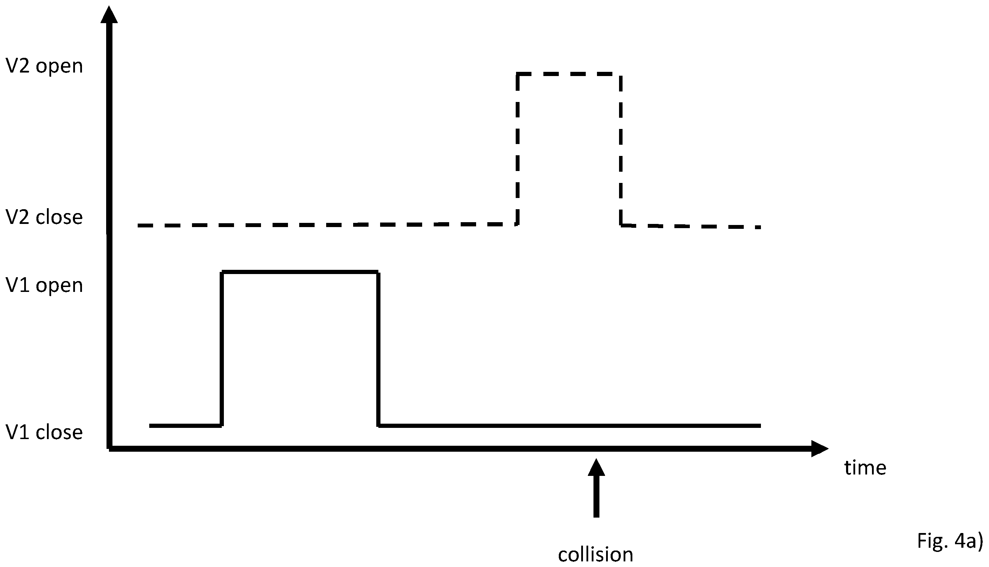

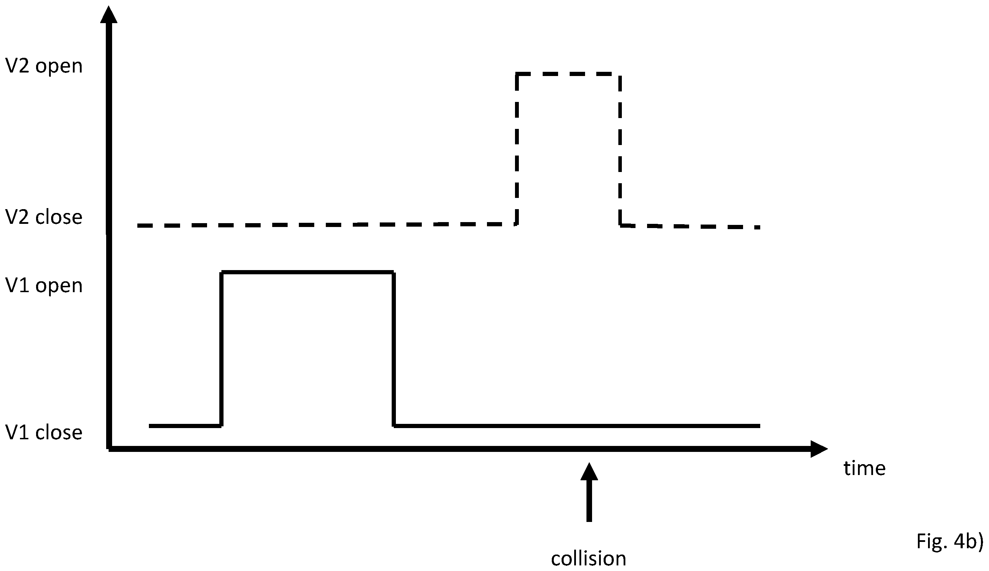

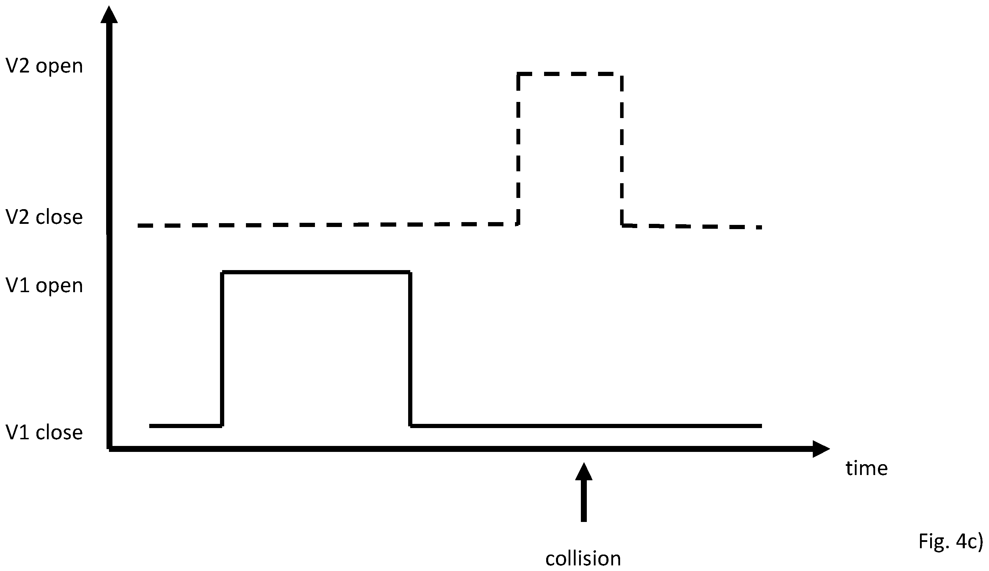

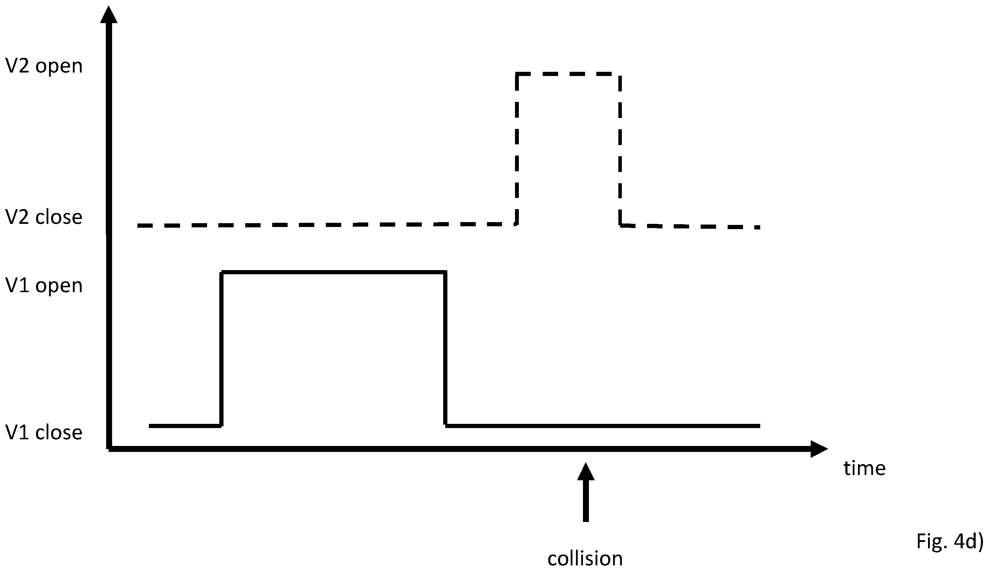

Figur 4 zeigt eine Folge von vier einzelnen schematischen Zeitverlaufsdiagrammen 4a) bis 4d), in denen jeweils mit der durchgezogenen Kurve der Öffnungs- und Schließvorgang des ersten Ventils 1 und mit der gestrichelten Kurve analog der Öffnungs- und Schließvorgang des zweiten Ventils 2 bezeichnet ist. Der erhöhte Kurventeil entspricht also jeweils der ersten/zweiten Einschaltzeit.

Die Figuren 4a) bis d) zeigen eine Abfolge von Druckbeaufschlagungspulsen der beiden Ventile 1 und 2 gemäß folgender Wertetabelle:

### Wertetabelle

| Frequenz [Hz] | 35 | 35 | 35 | 35 |
|---|---|---|---|---|
| Projektilgeschwindigkeit [m/s] | 4.3 | 5.4 | 7.3 | 10 |
| Öffnungszeitpunkt Ventil 1 [ms] | 0 | 0 | 0 | 0 |
| Schließzeitpunkt Ventil 1 [ms] | 9 | 10 | 11 | 13 |
| Öffnungszeitpunkt Ventil 2 [ms] | 17 | 17 | 17 | 17 |
| Schließzeitpunkt Ventil 2 [ms] | 23 | 23 | 23 | 23 |
| Aufschlagzeitpunkt [ms] | 21.6 | 21.3 | 21 | 21.1 |

Konkret wird nach einer jeweiligen (eingezeichneten) Kollision zwischen dem Projektil 8 und dem Applikator 6 einerseits durch den Impulsaustausch dabei und andererseits durch die pneumatische Beaufschlagung während der verbleibenden Rests der zweiten Einschaltzeit das Projektil in Rückrichtung beschleunigt, wobei es aber nicht bis zum distalen Ende der maximal möglichen Bewegungsstrecke bewegt wird, sondern durch den mit der folgenden ersten Einschaltzeit einsetzenden pneumatischen Gegendruck (bei wegfallendem beschleunigenden Druck) abgebremst wird. Dadurch wird das Projektil letztlich in seiner Bewegungsrichtung umgekehrt, bevor es das distale Ende erreicht und wieder in der Hinrichtung beschleunigt. Diese Beschleunigung endet mit dem jeweiligen Ende der ersten Einschaltzeit, wobei das Projektil während der dann folgenden Abstandszeit noch näherungsweise kräftefrei weiter fliegt, um ungefähr zeitgleich mit dem Beginn der folgenden zweiten Einschaltzeit (oder auch etwas früher oder später) mit dem Applikator 6 zu kollidieren. Dann folgt derselbe Zyklus ein weiteres Mal.

Der Unterschied zwischen den vier Einzeldarstellungen besteht in den jeweils länger werdenden Zeitdauern der ersten Einschaltzeiten und damit den kleiner werdenden Abstandszeiten zu den zweiten Einschaltzeiten. Demzufolge nimmt die zurückgelegte Teilstrecke der maximal möglichen Bewegungsstrecke von Figur 4 a) bis d) zu. Da der beschleunigende Druck gleich bleibt, nimmt damit gleichzeitig auch die Kollisionsgeschwindigkeit bei der Kollision mit dem Applikator 6 zu. Durch Wahl verschiedener Abstandszeiten oder, hier nicht gezeigt, Überlappzeiten kann dabei die Kollisionsgeschwindigkeit zusätzlich beeinflusst werden.

Bei einer typischen Rohrlänge im Bereich um 145.5 mm lässt sich mit den Werten aus der Tabelle zeigen, dass offensichtlich bei einer Frequenz von 35 Hz wie in diesem Beispiel nur noch ein Teil der Rohrlänge ausgenutzt werden kann. Selbst wenn das Projektil die Kollisionsgeschwindigkeit von 4.3 m/s während der Hin- und Herbewegung innerhalb des Rohrs konstant beibehalten würde, käme es nur in einer halben Umlaufzeit auf eine Strecke von 60 mm Gesamtlänge, was deutlich weniger als die tatsächliche Rohrlänge ist. Mit der bisherigen Technologie sind also solch hohe Kollisionsfrequenzen bei gleichzeitig vergleichsweise geringen Kollisionsgeschwindigkeiten nicht möglich.

Genau genommen zeigen die Figuren 4a) bis d) die elektrischen Steuerzeiten der beiden Ventile 1 und 2, also die Ausgangssignale der Steuerung 54. Die Ventile 1 und 2 sind federunterstützte Magnetventile, die rein magnetisch öffnen und durch die Kraft der dabei gespannten Feder schließen, wenn der Magnet nicht mehr beaufschlagt wird. Die Bewegungen des Ventilkörpers sind dementsprechend gegenüber den dargestellten Steuersignalen etwas verzögert, und zwar um schätzungsweise 4 ms beim Öffnen und 2 ms beim Schließen. Die Abstandszeiten sind also tatsächlich ungefähr 2 ms längerer als dargestellt.

Bei einem sogenannten Pilotventil mit pneumatischer Unterstützung beim Öffnen wäre die Situation qualitativ vergleichbar.

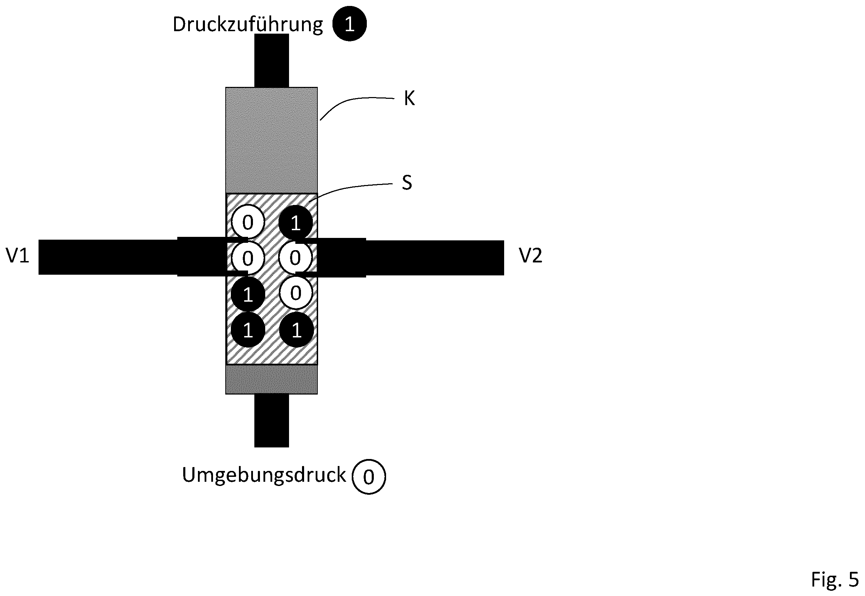

Natürlich kann man bei einem anderen Ausführungsbeispiel mit einem "Kombinationsventil" ganz ähnliche Verhältnisse erzeugen wie in Figur 4 in den Diagrammen a) bis d) dargestellt, wobei dann aber die Überlappzeit einen anderen Schaltzustand des Ventils bedeuten würde. Ein solches Kombinationsventil ist in Figur 5 schematisch dargestellt. Dabei bezeichnet der Buchstabe K das Kombinationsventil, das dementsprechend die beiden Ventile 1 und 2 aus den Figuren 1 und 2 ersetzt. Rechts und links sind zwei Leitungen V1 und V2 dargestellt, von denen V1 einen Anschluss an die linke Seite (gemäß Figur 2) des Projektilführungsrohrs 7, z. B. über das Kanalstück 22 (analog zu dem ersten Ventil 1) bedeutet. Dementsprechend bedeutet die rechte Leitung V2 einen Anschluss an die rechte Seite des Projektilführungsrohrs 7 (analog zu dem zweiten Ventil 2), also z. B. über das Kanalstück 25.

Die obere Leitung ist in Figur 5 mit dem Stichwort "Druckzuführung" und dem Symbol "1" (nicht zu verwechseln mit dem Bezugszeichen 1) für das erste Ventil bezeichnet; analog ist der untere Leitungsanschluss mit dem Stichwort "Umgebungsdruck" und dem figureninternen Symbol "0" bezeichnet, bedeutet also eine Belüftungsöffnung.

In dem Kombinationsventil K gibt es einen symbolisch dargestellten Schieber S, der in der vertikalen Richtung (bezogen auf Figur 5) zwischen vier verschiedenen Schaltpositionen verschoben werden kann. In der obersten ist, wie die Figur 5 verdeutlicht, der Anschluss V1 belüftet und der Anschluss V2 mit dem pneumatischen Versorgungsdruck beaufschlagt, in der dritten von oben umgekehrt und in der gerade aktiv geschalteten zweiten Position von oben sind beide Anschlüsse V1 und V2 belüftet. Schließlich zeigt die unterste Position eine gleichzeitige Druckbeaufschlagung beider Anschlüsse V1 und V2.

Man könnte sich also ein in dieser oder ähnlicher Weise aufgebautes Kombinationsventil K anstelle der beiden einzelnen Ventile 1 und 2 aus dem Ausführungsbeispiel in den Figuren 1 und 2 vorstellen, wobei die übrigen Erläuterungen und insbesondere die Figuren 3 und 4 sinngemäß auch dafür gelten.

Wegen der Steuerungsmöglichkeit der Aufschlaggeschwindigkeit des Projektils 8 allein über den Schaltbetrieb der beiden Ventile 1 und 2 läuft der Pneumatikkompressor 53 (Figur 3) bei einer vorgegebenen festen Betriebsfrequenz, in der er ein Wirkungsgradmaximum hat. Außerdem kann der Pneumatikkompressor bei einer vorgegebenen Betriebsfrequenz besonders wirksam schwingungs- und geräuschgedämmt werden.

Grundsätzlich kann die Steuereinrichtung 54 die Aufschlaggeschwindigkeit und auch den Zeitabstand zwischen den Kollisionen zwischen dem Projektil 8 und dem Applikator 6 von einem zu nächsten Einzelvorgang verändern. Sie kann also deutlich schneller und variabler die Stoßphysik beeinflussen und ist insbesondere nicht an periodische Vorgänge gebunden.

## Patentansprüche

1. Gerät zur Behandlung des menschlichen oder tierischen Körpers mit mechanischen Druckwellen, welches Gerät aufweist:
- ein Projektil (8), das in dem Gerät entlang der Bewegungsstrecke geführt ist,
- einen Applikator (6) an einem Ende und einem Anschlag an einem anderen Ende der Bewegungsstrecke,
- eine pneumatische Einrichtung zu Beaufschlagung des Projektils (8) mit pneumatischem Druck zwecks einer Bewegung entlang der Bewegungsstrecke,
wobei das Projektil (8) zu einem Aufschlagen auf den Applikator (6) zur Erzeugung der mechanischen Druckwellen ausgelegt ist,
welche pneumatische Einrichtung eine Doppelventileinrichtung (1, 2) zur Beaufschlagung des Projektils (8) mit pneumatischem Druck in Richtung zu dem Applikator (6) hin während einer ersten Einschaltzeit und zur Beaufschlagung des Projektils (8) mit pneumatischem Druck in der Rückrichtung während einer zweiten Einschaltzeit sowie eine Steuereinrichtung (54) zur Ansteuerung der Doppelventileinrichtung (1,2) aufweist,
**dadurch gekennzeichnet, dass** das Gerät dazu ausgelegt ist, nach einer teilweisen Rückbewegung in einer zweiten Einschaltzeit diese zweite Einschaltzeit zu beenden, eine erste Einschaltzeit zu beginnen und durch die Beaufschlagung des Projektils (8) mit pneumatischem Druck nach nur einem Teil der Bewegungsstrecke und vor dem Ende mit dem Anschlag die Bewegung des Projektils (8) von einer Rückbewegung in eine Hinbewegung umzudrehen.

2. Gerät nach Anspruch 1, bei dem die Doppelventileinrichtung (1,2) ein erstes Ventil zur Beaufschlagung des Projektils (8) mit pneumatischem Druck in Richtung zu dem Applikator (6) hin und ein zweites Ventil zur Beaufschlagung des Projektils (8) mit pneumatischem Druck in der Rückrichtung aufweist, die vorzugsweise unabhängig voneinander von der Steuereinrichtung (54) ansteuerbar sind.

3. Gerät nach Anspruch 1, bei dem die Doppelventileinrichtung (1,2) ein "Kombinationsventil " aufweist, das abhängig von der Ansteuerung durch die Steuereinrichtung (54) einen ersten Schaltzustand zur Beaufschlagung des Projektils (8) mit pneumatischem Druck in Richtung zu dem Applikator (6) hin oder einen zweiten Schaltzustand zur Beaufschlagung des Projektils (8) mit pneumatischem Druck in der Rückrichtung einnimmt, wobei in jedem dieser Schaltzustände der in dem jeweils anderen Schaltzustand zur Beaufschlagung des Projektils (8) verwendete pneumatische Anschluss durch das Kombinationsventil belüftet wird

4. Gerät nach Anspruch 2, bei dem mindestens eines der beiden Ventile ein Zweiwegeventil ist, das ein pneumatisches Volumen zwischen sich und dem Projektil (8) in einer ersten Schaltstellung während der jeweiligen Einschaltzeit zur Beaufschlagung des Projektils (8) mit pneumatischen Druck beaufschlagt und das in einer zweiten Schaltstellung dieses pneumatische Volumen belüftet.

5. Gerät nach einem der vorstehenden Ansprüche, dazu ausgelegt, eine Aufschlaggeschwindigkeit des Projektils (8) beim Aufschlagen auf den Applikator (6) zu steuern und dabei den vor dem Umdrehen der Bewegung des Projektils (8) von dem Projektil (8) zurückgelegten Teil der Bewegungsstrecke zu variieren.

6. Gerät nach einem der vorgenannten Ansprüche, dazu ausgelegt, die erste und die zweite Einschaltzeit in einer Überlappzeit überlappen zu lassen.

7. Gerät nach einem der vorstehenden Ansprüche, bei dem die Steuereinrichtung (54) dazu ausgelegt ist, in verschiedenen Steuerungszuständen mit der Überlappzeit Null eine Abstandszeit zwischen einer ersten und einer zweiten Einschaltzeit zu variieren.

8. Gerät nach einem der vorstehenden Ansprüche, dazu ausgelegt, dass bei der Steuerung der an der Doppelventileinrichtung (1,2) anstehende pneumatische Versorgungsdruck für die Beaufschlagung unverändert bleibt.

9. Gerät nach einem der vorstehenden Ansprüche, bei dem die pneumatische Einrichtung einen Pneumatikkompressor (53) aufweist, wobei das Gerät dazu ausgelegt ist, den Kompressor im eingeschalteten Zustand bei verschiedenen Steuerungszuständen mit unterschiedlichen Aufschlaggeschwindigkeiten des Projektils (8) bei gleicher Drehzahl laufen zu lassen, vorzugsweise grundsätzlich im eingeschalteten Zustand bei immer der gleichen Drehzahl laufen zu lassen.

10. Gerät nach einem der vorstehenden Ansprüche, bei welchem das Projektil (8) mit einem Aufschlagimpuls von zwischen 2 gm/s und 300 gm/s beim Aufschlagen auf den Applikator (6) bewegt werden kann.

11. Gerät nach einem der vorstehenden Ansprüche, dazu ausgelegt, in einem iterativen Betriebszustand mit unmittelbar aufeinanderfolgenden Hinbewegung des Projektils (8) zum Aufschlag auf den Applikator (6) und Rückbewegungen die Aufschlaggeschwindigkeit von einer zur nächsten solchen kombinierten Hin- und Rückbewegung zu verändern.

12. Gerät nach einem der vorstehenden Ansprüche mit einer Messeinrichtung (31) zur Erfassung eines Durchtritts des Projektils (8) an einer Stelle der Bewegungsstrecke, welche Messeinrichtung (31) mit der Steuereinrichtung (54) gekoppelt ist.

## Claims

1. Apparatus for treatment of the human or animal body with mechanical pressure waves, the apparatus comprising:
- a projectile (8) guided in the apparatus along the movement path,
- an applicator (6) at one end and a stop at another end of the movement path,
- pneumatic means for application of pneumatic pressure to the projectile (8) for the purpose of movement along the movement path,
wherein the projectile (8) is adapted for striking onto the applicator (6) for generating the mechanical pressure waves,
which pneumatic means has a double valve means (1, 2) for application of pneumatic pressure to the projectile (8) in the direction towards the applicator (6) during a first activation time and for application of pneumatic pressure to the projectile (8) in the reverse direction during a second activation time and a control means (54) for controlling the double valve means (1, 2), **characterized in that** the apparatus is adapted, after a partial return movement in a second activation time, to end this second activation time, to start a first activation time and, by the application of pneumatic pressure to the projectile (8) after only a part of the movement path and before the end with the stop, to reverse the movement of the projectile (8) from a return movement into a forward movement.

2. Apparatus according to claim 1, in which the double valve means (1, 2) has a first valve for application of pneumatic pressure to the projectile (8) in the direction towards the applicator (6) and a second valve for application of pneumatic pressure to the projectile (8) in the reverse direction, which valves can preferably be controlled independently of one another by the control means (54).

3. Apparatus according to claim 1, in which the double valve means (1, 2) has a "combination valve" which, depending on the control by the control means (54), assumes a first switching state for application of pneumatic pressure to the projectile (8) in the direction towards the applicator (6) or a second switching state for application of pneumatic pressure to the projectile (8) in the reverse direction, wherein in each of these switching states the pneumatic connection used in the respectively other switching state for application of pneumatic pressure to the projectile (8) is ventilated by the combination valve.

4. Apparatus according to claim 2, in which at least one of the two valves is a two-way valve which applies pneumatic pressure to a pneumatic volume between itself and the projectile (8) in a first switching position during the respective activation time for application of pneumatic pressure to the projectile (8) and which ventilates this pneumatic volume in a second switching position.

5. Apparatus according to one of the preceding claims, adapted to control an impact speed of the projectile (8) upon impact onto the applicator (6) and in the process to vary the part of the movement path covered by the projectile (8) before the rotation of the movement of the projectile (8).

6. Apparatus according to one of the preceding claims, adapted to allow the first and the second activation time to overlap in an overlap time.

7. Apparatus according to one of the preceding claims, in which the control means (54) is adapted to vary in different control states with the overlap time zero a separation time between a first and a second activation time.

8. Apparatus according to one of the preceding claims, adapted so that during the control the pneumatic supply pressure applied to the double valve means (1, 2) remains unchanged for the application of pressure.

9. Apparatus according to one of the preceding claims, wherein the pneumatic means comprises a pneumatic compressor (53), wherein the apparatus is adapted to allow the compressor in the activated state to run at different control states with different impact speeds of the projectile (8) at the same rotational frequency, preferably in principle in the activated state to run at always the same rotational frequency.

10. Apparatus according to one of the preceding claims, wherein the projectile (8) can be moved with an impact pulse of between 2 gm/s and 300 gm/s upon impact onto the applicator (6).

11. Apparatus according to one of the preceding claims, adapted to vary, in an iterative operating state with directly successive forward movements of the projectile (8) for impact onto the applicator (6) and return movements, the impact speed from one to the next such combined forward and return movement.

12. Apparatus according to one of the preceding claims, having a measuring means (31) for detecting a passage of the projectile (8) at a point of the movement path, which measuring means (31) is coupled to the control means (54).

## Revendications

1. Appareil pour le traitement du corps humain ou animal avec des ondes de pression mécaniques, l'appareil comprenant :
- un projectile (8) guidé dans l'appareil le long de la trajectoire de déplacement,
- un applicateur (6) à une extrémité et une butée à une autre extrémité de la trajectoire de déplacement,
- un dispositif pneumatique pour appliquer une pression pneumatique au projectile (8) en vue d'un déplacement le long de la trajectoire de déplacement,
le projectile (8) étant conçu pour percuter l'applicateur (6) afin de générer les ondes de pression mécaniques,
le dispositif pneumatique comprenant un dispositif à double soupape (1, 2) pour appliquer une pression pneumatique au projectile (8) en direction de l'applicateur (6) pendant un premier temps de mise en marche et pour appliquer une pression pneumatique au projectile (8) dans le sens inverse pendant un deuxième temps de mise en marche, ainsi qu'un dispositif de commande (54) pour commander le dispositif à double soupape (1, 2), **caractérisé en ce que** l'appareil est conçu pour terminer ce deuxième temps de mise en marche après un déplacement inverse partiel dans un deuxième temps de mise en marche, pour commencer un premier temps de mise en marche et pour inverser le déplacement du projectile (8) d'un déplacement inverse à un déplacement aller par l'application d'une pression pneumatique au projectile (8) après seulement une partie de la trajectoire de déplacement et avant la fin avec la butée.

2. Appareil selon la revendication 1, dans lequel le dispositif à double soupape (1, 2) comprend une première soupape pour appliquer une pression pneumatique au projectile (8) en direction de l'applicateur (6) et une deuxième soupape pour appliquer une pression pneumatique au projectile (8) dans le sens inverse, lesquelles peuvent être commandées de préférence indépendamment l'une de l'autre par le dispositif de commande (54).

3. Appareil selon la revendication 1, dans lequel le dispositif à double soupape (1, 2) comprend une « soupape combinée » qui, en fonction de la commande par le dispositif de commande (54), adopte un premier état de commutation pour appliquer une pression pneumatique au projectile (8) en direction de l'applicateur (6) ou un deuxième état de commutation pour appliquer une pression pneumatique au projectile (8) dans le sens inverse, le raccord pneumatique utilisé dans l'autre état de commutation respectif pour appliquer une pression au projectile (8) étant ventilé par la soupape combinée dans chacun de ces états de commutation.

4. Appareil selon la revendication 2, dans lequel au moins l'une des deux soupapes est une soupape à deux voies qui applique une pression pneumatique à un volume pneumatique entre elle-même et le projectile (8) dans une première position de commutation pendant le temps de mise en marche respectif pour appliquer une pression pneumatique au projectile (8) et qui ventile ce volume pneumatique dans une deuxième position de commutation.

5. Appareil selon l'une quelconque des revendications précédentes, conçu pour commander une vitesse de percussion du projectile (8) lors de la percussion sur l'applicateur (6) et pour faire varier la partie de la trajectoire de déplacement parcourue par le projectile (8) avant l'inversion du déplacement du projectile (8).

6. Appareil selon l'une quelconque des revendications précédentes, conçu pour laisser le premier et le deuxième temps de mise en marche se chevaucher dans un temps de chevauchement.

7. Appareil selon l'une quelconque des revendications précédentes, dans lequel le dispositif de commande (54) est conçu pour faire varier, dans différents états de commande avec le temps de chevauchement nul, un temps d'écart entre un premier et un deuxième temps de mise en marche.

8. Appareil selon l'une quelconque des revendications précédentes, conçu pour que, lors de la commande, la pression d'alimentation pneumatique régnant sur le dispositif à double soupape (1, 2) reste inchangée pour la sollicitation.

9. Appareil selon l'une quelconque des revendications précédentes, dans lequel le dispositif pneumatique comprend un compresseur pneumatique (53), l'appareil étant conçu pour faire fonctionner le compresseur à l'état mis en marche dans différents états de commande avec différentes vitesses de percussion du projectile (8) à la même vitesse de rotation, de préférence pour le faire fonctionner en principe à l'état mis en marche toujours à la même vitesse de rotation.

10. Appareil selon l'une quelconque des revendications précédentes, dans lequel le projectile (8) peut être déplacé avec une impulsion de percussion comprise entre 2 gm/s et 300 gm/s lors de la percussion sur l'applicateur (6).

11. Appareil selon l'une quelconque des revendications précédentes, conçu pour modifier, dans un état de fonctionnement itératif avec des déplacements aller directement successifs du projectile (8) pour la percussion sur l'applicateur (6) et des déplacements inverses, la vitesse de percussion d'un tel déplacement aller et inverse combiné au suivant.

12. Appareil selon l'une quelconque des revendications précédentes, comprenant un dispositif de mesure (31) pour détecter un passage du projectile (8) à un endroit de la trajectoire de déplacement, lequel dispositif de mesure (31) est couplé au dispositif de commande (54).
